(19) Europäisches Patentamt
European Patent Office
Office européen des brevets

(11) Publication number: 0 136 569
A2

# (12) EUROPEAN PATENT APPLICATION

(21) Application number: 84110423.5

(22) Date of filing: 01.09.84

(51) Int. Cl.[4]: C 07 D 311/36
A 61 K 31/35

(30) Priority: 05.09.83 JP 163914/83

(43) Date of publication of application: 10.04.85 Bulletin 85/15

(84) Designated Contracting States: BE CH DE FR GB IT LI NL SE

(71) Applicant: Takeda Chemical Industries, Ltd.
27, Doshomachi 2-chome Higashi-ku
Osaka-shi Osaka, 541(JP)

(72) Inventor: Yamazaki, Iwao
9-21, Hibarigaokayamate 1-chome
Takarazuka Hyogo 665(JP)

(72) Inventor: Sawa, Yoichi
19-11, Sengokuhigashi-machi
Kadoma Osaka 571(JP)

(74) Representative: von Kreisler, Alek, Dipl.-Chem. et al,
Deichmannhaus am Hauptbahnhof
D-5000 Köln 1(DE)

(54) **Isoflavone derivatives, their production and use.**

(57) Novel isoflavone derivatives of the formula:

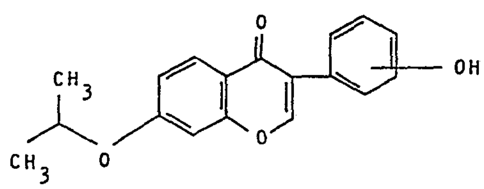

exhibit mild estrogenic activity, and are of value as an agent for prevention and treatment of osteoporosis.

EP 0 136 569 A2

Isoflavone derivatives, Their Production and Use

This invention relates to novel isoflavone derivatives which are of value as medicines, and method for producing said derivatives.

More particularly, this invention relates to a novel isoflavone derivative (4H-1-benzopyran-4-one derivative) of the formula:

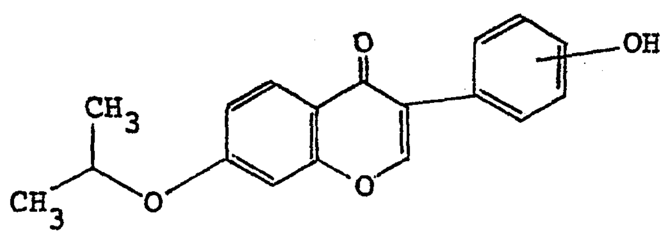

[I]

which exhibits mild estrogenic activity, and is of value as an agent for prevention and treatment of osteoporosis, and its production.

Osteoporosis is a disease condition or illness which occurs frequently in postmenopausal females, particularly those in their sixties, and wherein the quantitative loss of bones progresses beyond a certain limit to thereby present some symptoms or risk manifestations. Among its main clinical manifestations are kyphosis, low back pain, and fractures of femoral neck, lower end of the radius, ribs, upper end of the humerus, etc. While the causative factors are variegated, including endocrine disorder and nutritional disorder, apparently the most important cause is a decreased secretion of estrogen due to hypoovarianism in females during the

postmenopausal period. Therefore, of all the therapeutic agents for osteoporosis, the theoretically most effective drugs are estrogen preparations. However, the estrogens so far available are so strong in effect as to cause side effects such as genital bleeding, mastodynia, hepatic disorder, and, for this reason, have not been used recently on as many occasions as in the past. There are other types of therapeutic agents such as calcitonin, vitamin D and calcium preparations, which however are disadvantageous in that they are either only indefinitely effective or ineffective when administered by the oral route.

Therefore, for the prevention or treatment of osteoporosis, it has been demanded a drug which would display a milder estrogenic activity than the conventional estrogens in oral administration and be free from side effects which are encountered with the known estrogens.

The present inventors conducted an intensive research for developing such a drug and found that the 4H-1-benzopyran-4-one derivative of the formula [I] has a mild estrogenic activity in oral administration and does not cause those side effects which are caused by the conventional estrogens.

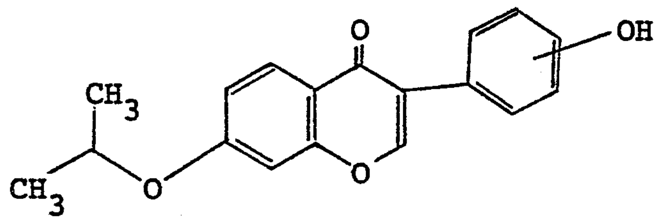

[I]

Therefore, it is an object of this invention to provide a novel 4H-1-benzopyran-4-one derivative of the formula [I] which is of value as a drug.

It is another object to provide a new method for producing said derivative.

It is still another object to provide a pharmaceutical preparation for prevention or treatment of osteoporosis which contains a 4H-1-benzopyran-4-one derivative of the formula [I] as an active component.

The compound [I] according to this invention is produced by isopropylating a hydroxy compound [II] with an isopropyl halide [III] in accordance with for example the following reaction formula:

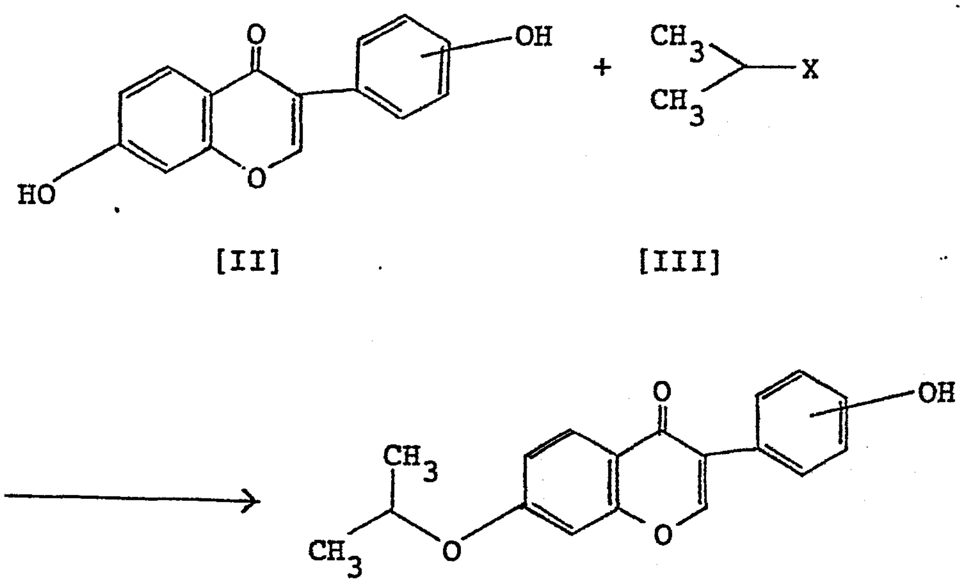

[II] [III]

[I]

wherein X is a halogen atom such as chlorine, bromine, iodine.

The starting material [II] used in accordance with this invention can be produced, for example by the method of E. M. Gaydou et al (Bull. Soc. Chim. Fr., 1978, II-43), i.e. by demethylating a 7-hydroxy-3-methoxyphenyl-4H-1-benzopyran-4-one, and the hydroxy group may be present in any substitutable position of the phenyl group. As examples of starting material [III], there may be mentioned isopropyl chloride, isopropyl bromide and isopropyl iodide.

Generally, the reaction according to this invention proceeds advantageously in the presence of an inert solvent. The solvent may be any solvent that will not interfere with the contemplated reaction and includes, among others, alcohols such as methanol, ethanol, isopropyl alcohol, ethers such as tetrahydrofuran, dioxane, diethyl ether, halogenated hydrocarbons such as dichloromethane, dichloroethane, chloroform, petroleum ether, toluene, xylene, dimethylformamide,

pyridine, aldehyde collidine, water, etc., as well as mixtures thereof.

The reaction proceeds advantageously in the presence of a deacidifying agent, such as an alkali hydrogen carbonate (e.g., sodium hydrogen carbonate and potassium hydrogen carbonate), an alkali hydroxide (e.g., sodium hydroxide and potassium hydroxide), an alkali carbonate (e.g., sodium carbonate and potassium carbonate), a sodium amide, sodium hydride, an alkali metal (e.g., sodium and potassium), a sodium alcoholate (e.g., sodium methylate and sodium ethylate), and an organic base (e.g., pyridine, aldehyde collidine, dimethylaniline and triethylamine).

The temperature and other conditions may be selected in an adequate manner.

One of the compounds [I] according to this invention was tested for its estrogenic activity and toxicity and the following results were obtained.

Test Example 1

Estrogenic activity of 7-isopropoxy-3-(4-hydroxyphenyl)-4H-1-benzopyran-4-one (hereinafter referred to as compound A) in young oophorectomized rats

Sprague-Dawley rats, 33 days old and 11 days after oophorectomy for elimination of endogenous estrogenic activity, were used in groups of 6 to 7 animals. Compound A was suspended in a 1% aqueous solution of hydroxypropyl-cellulose and administered orally for 3 days, while as a representative example of the conventional estrogen drug, estrone was dissolved in sesame oil and administered subcutaneously for 3 days. On the fourth day, each animal was autopsied and its uterine wet weight was recorded. As shown in Table 1, compound A at the daily dose levels of 100 mg/kg and 400 mg/kg produced uterine weight increasing effect with a dose-response curve of moderate gradient. In contrast, estrone showed uterine weight increasing effect with a dose-response curve of steep gradient.

Table 1

| Compound | Daily dose (mg/kg) | Number of animals | Uterine wet weight (mg ± S.D.) |
|---|---|---|---|
| Compound A | 0 (control group) | 7 | 31.1 ± 1.1 |
| | 6.25 | 7 | 32.6 ± 1.2 |
| | 25 | 7 | 39.2 ± 2.3 |
| | 100 | 7 | 56.3 ± 3.7* |
| | 400 | 6 | 83.0 ± 6.9* |
| Estrone | 0.0025 | 7 | 121.1 ± 7.0* |
| | 0.005 | 7 | 200.5 ± 14.3* |
| | 0.01 | 7 | 247.2 ± 14.6* |

*: Significant as compared with control group (P<0.01)

Test Example 2

Acute toxicity

Five-week-old ICR mice and 5-week-old Sprague-Dawley rats were respectively used in groups of 10 males and 10 females, and suspensions of compound A in olive oil were administered orally [2,500, 5,000 and 10,000 mg/kg] or subcutaneously [1,250, 2,500 and 5,000 mg/kg]. The animals were kept under observation for 14 days. None of the groups showed deaths nor toxic symptoms attributable to compound A and therefore, $LD_{50}$ values could not be calculated.

The daily dosage of the compound of the formula [I] for human beings is generally about 1 to 50 mg/kg and preferably about 5 to 30 mg/kg for oral administration, and about 200 to 600 mg can be orally taken daily, once a day or, if necessary, in 2 to 3 divided doses. The compounds are preferably formulated into such dosage forms as tablets, capsules, etc. by the established pharmaceutical procedure. Such tablets and capsules can be prepared using suitable

excipients such as lactose, starch, binders such as hydroxypropylcellulose, and lubricants such as magnesium stearate. The tablets may be sugar-coated, if necessary.

Example 1

A mixture of 54 g of 7-hydroxy-3-(4-hydroxyphenyl)-4H-1-benzopyran-4-one, 350 ml of dimethylformamide, 52 g of isopropyl bromide and 52.5 g of anhydrous sodium carbonate is stirred thoroughly with heating at 80°C for 5 hours and then poured into 1.8 liters of cold water. The resulting crystalline precipitate is collected by filtration and recrystallized from dichloroethane to give 7-isopropoxy-3-(4-hydroxyphenyl)-4H-1-benzopyran-4-one as white crystals. mp 184-185°C.

Elemental analysis

Calcd. for $C_{18}H_{16}O_4$:
(%) C, 72,96; H, 5.44

Found (%) C, 72.76; H, 5.38

NMR (DMSO-$d_6$)$\delta$: 1.33(6H,d,$CH_3$x2), 4.55-4.95 (1H,m,CH), 6.8-7.5(6H,m,aromatic H), 8.28 (1H,s,$C_2$H), 9.5(1H,bs,OH)

In the same manner as above, there are obtained the following compounds.

7-Isopropoxy-3-(3-hydroxyphenyl)-4H-1-benzopyran-4-one

NMR (DMSO-$d_6$)$\delta$: 1.35(6H,d,$CH_3$x2), 4.5-5.0(1H,m,CH), 6.7-7.5(6H,m,aromatic H), 8.04(1H,d,$C_5$-H), 8.30(1H,s,$C_2$-H), 9.5(1H,bs,OH)

7-Isopropoxy-3-(2-hydroxyphenyl)-4H-1-benzopyran-4-One

NMR (DMSO-$d_6$)$\delta$: 1.34(6H,d,$CH_3$x2), 4.5-5.0 (1H,m,CH), 6.8-7.4(6H,m,aromatic H), 7.95(1H,d,$C_5$-H), 8.20(1H,s,$C_2$-H), 9.4(1H,bs,OH)

Example 2 Tablets

| | | |
|---|---|---|
| I) | 7-Isopropoxy-3-(4-hydroxyphenyl)-4H-1-benzopyran-4-one | 200 g |
| II) | Lactose | 15 g |

| | | |
|---|---|---|
| III) | Starch | 45 g |
| IV) | Carboxymethylcellulose calcium | 10 g |
| V) | Magnesium stearate | 1 g |

The above components I) through V) are admixed to prepare 1000 uncoated tablets with a diameter of 8.5 mm.

Example 2 Capsules

| | | |
|---|---|---|
| I) | 7-Isopropoxy-3-(4-hydroxyphenyl)-4H-1-benzopyran-4-one | 200 g |
| II) | Lactose | 40 g |
| III) | Starch | 50 g |
| IV) | Hydroxypropylcellulose | 7 g |
| V) | Magnesium stearate | 3 g |

The above components I) through V) are admixed and filled into 1000 No. 1 capsules.

What is claimed is:

1. A compound of the formula

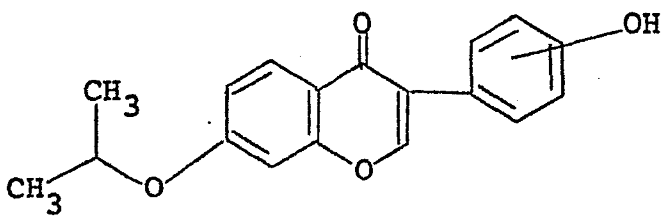

2. The compound according to claim 1, which is 7-isopropoxy-3-(4-hydroxyphenyl)-4H-1-benzopyran-4-one.

3. A pharmaceutical composition suitable for prevention or treatment of osteoporosis which comprises, as an active ingredient, an effective amount of compound as claimed in any one of claims 1 and 2 in association with a pharmaceutically acceptable carrier, excipient or diluent therefor.

4. A compound as claimed in any one of claims 1 and 2 or a pharmaceutical composition as claimed in claim 3 for use in therapeutical treatment of a female.

5. A method for producing a compound of the formula

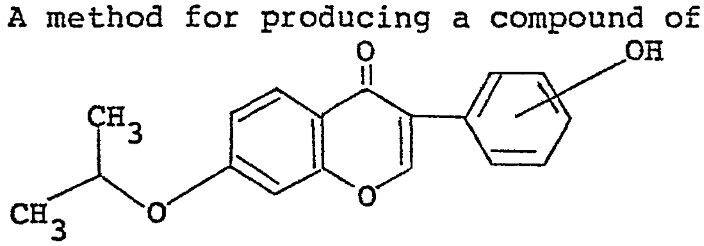

which comprises isopropylating a compound of the formula

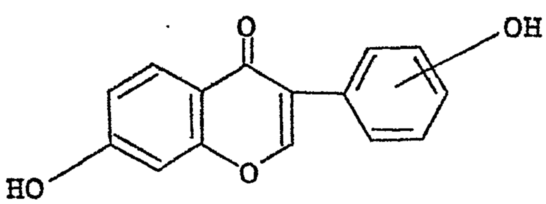

6. The method according to claim 5, wherein 7-isopropoxy-3-(4-hydroxyphenyl)-4H-1-benzopyran-4-one is produced by isopropylating 7-hydroxy-3-(4-hydroxyphenyl)-4H-benzopyran-4-one.